# EUROPEAN PATENT APPLICATION

(11) **EP 3 495 469 A1**
(43) Date of publication of application: **12.06.2019**
(21) Application number: 17206036.0
(22) Date of filing: 07.12.2017
(51) Int. Cl.: C12N 1/00, C12N 1/20, C12N 1/36, C07K 14/255, A61K 39/112, A61K 39/39

(54) **VACCINE COMPOSITIONS II**

(71) Applicant: Prokarium Limited, Keele Science Park Keele Staffordshire ST5 5SP (GB)
(72) Inventor: CRANENBURGH, Rocky, Keele Staffordshire ST5 5SP (GB)
(74) Representative: Gill Jennings & Every LLP

(57) **Abstract**

The present invention provides a genetically engineered non-natural bacterium comprising inactivating mutations in one or more genes selected from *ptlA, ptlB, cdtB* and *ttsA,* wherein the bacterium is derived from *Salmonella* spp, in particular Salmonella enterica, such as *S. enterica* Typhi, *S. enterica* Paratyphi A, *S. enterica* Paratyphi B or *S. enterica* Paratyphi C. The one or more inactivating mutations may comprise the partial or complete deletion of the one or more genes selected from *ptlA, ptlB, cdtB* and *ttsA.* The non-natural bacterium of the invention is provided for use in the treatment or prevention of a disease, such as an infectious disease or cancer. For example the bacterium may be for use in vaccination against a disease, such as typhoid or paratyphoid enteric fever. The bacterium may also be used as an anti-cancer vaccine comprising suitable antigens.

<110>  Prokarium Limited

<120>  Vaccine Compositions II

<130>  JWJ02381EP

<160>  8

<170>  PatentIn version 3.5

<210>  1
<211>  729
<212>  DNA
<213>  Salmonella enterica

<400>  1
atgaaaaagt taatattctt aaccttatct atagttagct ttaataacta tgctgtagat      60

tttgtgtatc gtgtggactc aaccccgccg gacgttattt ttcgcgatgg gttttcacta     120

cttgggtata accgtaactt tcagcaattt attagtggaa ggtcatgtag tggtggaagt     180

agtgacagtc gctatattgc aacaacctca agtgttaatc aaacatatgc tatagccaga     240

gcgtactatt ctcgctcaac attcaaaggt aatttataca gatatcagat tcgtgcagat     300

aataatttct acagcttgct cccatccatc acctatctgg aaacgcaagg tggtcacttt     360

aatgcttatg aaaaaacgat gatgcgattg caaagagagt atgtttccac attatctatt     420

ttacccgaga atattcaaaa ggccgtggcg ctagtttatg atagcgcaac cggtctagta     480

aaggatggtg taagcacaat gaatgccagt tatttaggtt taagcactac gtctaatcct     540

ggtgtgatac cttttcttcc ggaaccgcag acgtataccc aacaacgaat tgatgcattc     600

ggcccattaa taagttcatg cttttcaata ggtagcgtat gtcattcaca tcgagggcaa     660

agagctgacg tatacaacat gtctttttat gatgcaagac ctgtaataga acttatactt     720

tctaaataa                                                             729


<210>  2
<211>  414
<212>  DNA
<213>  Salmonella enterica

<400>  2
atgtatatga gtaagtatgt acctgtttat acattattaa ttctcattta ttcttttaat      60

gccagcgctg agtggacagg agataatacg aacgcctact actcagacga agttatcagt     120

gaattacatg ttggtcagat agatactagt ccttattttt gcataaaaac ggttaaagct     180

aacggtagcg gtacaccagt tgttgcatgt gcggtatcaa agcagagcat atgggcgccc     240

tcctttaaag aacttcttga tcaggcaaga tatttttaca gtacagggca atccgtaagg     300

attcatgttc aaaaaaatat ctggacctat ccgctttttg ttaatacctt ttcagcaaat     360

gctcttgtgg gactatcatc gtgcagtgcg acacaatgct ttggacccaa gtaa 414

<210> 3
<211> 810
<212> DNA
<213> Salmonella enterica

<400> 3
atgaaaaaac ctgttttttt ccttctgacc atgatcatct gcagctatat ttcttttgcc 60

tgcgctaata tcagtgacta caaagttatg acctggaatc ttcagggctc ttcagcatct 120

acagaaagta aatggaatgt caatgtcaga cagcttttaa gtggtactgc cggtgtggat 180

attcttatgg tacaggaggc cggtgctgtt cccacctcag cggttcctac cggacgacat 240

attcagcctt ttggagtggg tattcccatt gatgaataca cctggaatct cggaaccacc 300

agccgtcagg atataagata tatctaccac tcggctattg atgttggagc acgccgtgtt 360

aatctggcaa tagtttccag acaaagagcg gataatgttt atgtcttgcg tccgacaact 420

gtcgcatctc gccccgtcat tggcatcgga ctgggtaatg atgtttttct gacagcgcac 480

gcactggcta gtggaggtcc ggatgctgca gctattgtca gggttaccat taattttttt 540

agacaacctc agatgcggca tttatcctgg tttcttgccg gggattttaa tcgcagccca 600

gacagacttg aaaatgacct gatgactgag catctggaac gagttgtagc cgtactcgca 660

cctacagaac ccacgcaaat tggcggtggt attttagatt atggggtcat tgtcgatcga 720

gcaccttatt cacaaagggt tgaagcatta cgtaatccac aactcgcttc tgatcattat 780

cccgtagcct ttttggcacg aagctgttaa 810

<210> 4
<211> 543
<212> DNA
<213> Salmonella enterica

<400> 4
atgactaaag atgaaatctt tgcagctatc cttagtagag aaggcggtta cgttgatcac 60

cctgacgaca ggggggggcc aacgcattgg ggtataaccc tgacgacggc ccgggctaat 120

ggctatatgg gggatatgcg gaatcttacc cgtaatcaag cattaaaaat ccttgaggca 180

gattattggt atggtccacg acttgatcag gttgccatta tctctcactc tattgcagct 240

gagctttgtg atactggtgt gaacatggga ccctcaatcc cgattaagta cttccagcgt 300

tggcttaatg tttttaatga tcagcaaaaa atttatccgg atttgatagc agatggtcag 360

attgggccac ggacgctttc ggcactaaca ttttttctct ctcaccggag agatgagggc 420

gaaatgatac ttatccgggc attgaattgt agtcagggac aacgctatct ggagttagct 480

gaaaagcgcc aggcgaatga atcatttgtt tatggatgga taaaggaacg ggtaagattg 540

taa 543

<210>  5
<211>  96
<212>  DNA
<213>  Artificial Sequence

<220>
<223>  Primer

<400>  5
tataatgaat ttgtgtttta ttaattattt taaacgtaca ggagagtagt ggtgcgcata        60

atgtatatta tgttaaatcc cttatgcgac tcctgc                                  96


<210>  6
<211>  98
<212>  DNA
<213>  Artificial Sequence

<220>
<223>  Primer

<400>  6
aactaataaa cgggctgttt aatttaatag gcaacatagg taagtttcat atttaacata        60

atatacatta tgcgcaccat ccgcttatta tcacttat                                98


<210>  7
<211>  98
<212>  DNA
<213>  Artificial Sequence

<220>
<223>  Primer

<400>  7
atatcattat atgctatcat cacaaaccgc tatagtggta agtaagggaa atttaacata        60

atatacatta tgcgcaccat ccgcttatta tcacttat                                98


<210>  8
<211>  98
<212>  DNA
<213>  Artificial Sequence

<220>
<223>  Primer

<400>  8
aacacatata tcattcagat aaagaagtaa tgctcgggag agtagatatt atttaacata        60

atatacatta tgcgcaccat ccgcttatta tcacttat                                98

## Description

### Field of the invention

This invention relates to genetically modified *Salmonella spp.* suitable for use as vaccine compositions, the use of said vaccines in the treatment and/or prevention of disease, and methods of producing said genetically modified *Salmonella spp.*

### Background of the invention

The use of the enteric pathogen *Salmonella enterica* as a live bacterial vector (LBV) has been studied extensively in experiments to express and deliver recombinant protein vaccines to cells. *Salmonella* LBVs are usually constructed by introducing into a host bacterium an expression cassette containing a promoter which regulates the expression of one or more genes encoding heterologous antigens from one or more pathogens. This cassette can either be chromosomally integrated in the host bacterial genome or carried by the host as an autonomously replicating multi-copy plasmid (Kochi et al. 2003, Expert Rev. Vaccines 2:31-43).

*Salmonella* LBVs are usually delivered orally for vaccine applications, either in a liquid form as an oral suspension or as an enterically coated capsule. The LBVs then enter the small intestine where they invade the intestinal epithelium via microfold (M) cells into Peyer's patches from where they are subsequently delivered to antigen-presenting cells (APCs) (Kotton & Hohmann 2004, Infect. Immun. 72: 5535-5547). Alternative delivery methods are available, and indeed often used, for example in anti-cancer applications, where the LBV may be injected systemically (e.g. intravenously) or directly into a tumour.

The standard approach for preparing *Salmonella enterica* LBVs from a host bacterium is to introduce specific, attenuating mutations into pathogenic genes of the host bacterium. These attenuating mutations reduce the survival of the host bacterium within the subject to prevent S. enterica-mediated side-effects.

The field has numerous examples of animal studies using host bacteria comprising attenuating genetic mutations. In general, these LBVs are sufficient to induce a prophylactic response against the heterologous antigen, but are not safe enough to be used in humans. This provides a false picture of the potential utility of LBVs for recombinant vaccine delivery.

Most commonly, attenuating mutations are made in genes involved in the aromatic amino acid (shikimate) biosynthesis pathway, such as *aroA* and *aroC.* However, although such attenuated LBVs are well tolerated in mice, these cause undesirable feverish symptoms and prolonged bacterial shedding in humans (Garmory et al. 2002, FEMS Microbiol. Rev. 26: 339-353). Conversely when a host bacterium is sufficiently attenuated for human use to deliver a heterologous antigen, the efficacy of the LBV is invariably disappointing (Garmory et al. 2002, FEMS Microbiol. Rev. 26: 339-353). This is because an improved LBV safety profile correlates with reduced immunogenicity. For example, extensive transposon-mediated insertional mutagenesis of the *Salmonella enterica* serovar Typhimurium genome failed to identify a mutant that was sufficiently immunogenic yet unable to cause disease in immunocompromised mice (Grant et al. 2016, Infect. Immun. 84: 989-997).

Therefore, there is a need to develop new methods for generating *Salmonella* LBVs to improve their safety profile but maintain sufficient immunogenicity to act as effective vaccine carriers or even vaccines in and of themselves.

The listing or discussion of an apparently prior-published document in this specification should not necessarily be taken as an acknowledgement that the document is part of the state of the art or is common general knowledge.

### Summary of the Invention

The present invention is based on the discovery that, in certain *Salmonella* species, inactivation of genes encoding factors essential for the production of typhoid toxin attenuates the bacterium whilst maintaining sufficient immunogenicity, thus enabling the use of the modified bacterium as a vaccine or vaccine carrier.

Thus, in a first aspect of the invention, there is provided a genetically engineered non-natural bacterium comprising inactivating mutations in one or more genes selected from *ptlA*, *ptlB*, *cdtB* and *ttsA,* wherein the non-natural bacterium is derived from *Salmonella* spp..

In a second aspect of the invention, there is provided the genetically engineered non-natural bacterium of the first aspect or a vaccine composition of the fifth aspect of the invention for use in the prevention or treatment of a disease, optionally wherein the disease is an infectious disease or cancer.

In a third aspect of the invention, there is provided a method of treatment or prevention of a disease, such as an infectious disease or cancer, comprising administering to a subject in need thereof a non-natural bacterium of the first aspect of the invention or a vaccine composition of the fifth aspect of the invention.

In a fourth aspect of the invention, there is provided the use of the non-natural bacterium of the first aspect of the invention or a vaccine composition of the fifth aspect of the invention in the manufacture of a medicament for treating or preventing a disease, optionally wherein the disease is an infectious disease or cancer.

In a fifth aspect of the invention, there is provided a vaccine composition comprising the non-natural bacterium of the first aspect and a pharmaceutically acceptable carrier.

In a sixth aspect of the invention, there is provided a method of manufacturing the non-natural bacterium according to the first aspect of the invention or a vaccine composition of the fifth aspect of the invention.

All documents referred to herein are incorporated herein by reference.

### Description of the Drawings

The invention is illustrated with reference to the following drawing, in which:

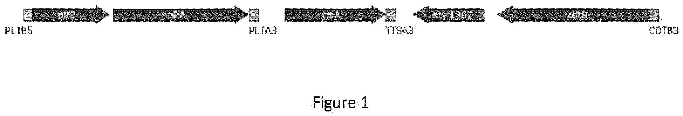

**Figure 1** shows the typhoid toxin locus from S. Typhi Ty2, showing the genes *pltB*, *pltA*, *ttsA* and *cdtB.* Homology of the DNA with the PCR primers PLTB5, PLTA3, TTSA3 and CDTB3 are shown in light grey. Homologous recombination using PCR products generated with primer PLTB5 plus either one of PLTA3, TTSA3 or CDTB3 will delete the intervening chromosomal DNA, creating typhoid toxin knock-out strains.

### Detailed description of the Invention

The invention is predicated on the discovery that the inactivation of genes associated with the production of the typhoid toxin in *Salmonella* spp. supresses the toxicity of the bacterium without eliminating immunogenicity. Thus, the non-natural bacteria of the invention provide effective carriers of heterologous antigens to be used as vaccines against infectious disease or self-antigens, where appropriate.

In a first aspect, the invention provides a genetically engineered non-natural bacterium comprising inactivating mutations in one or more genes selected from *ptlA*, *ptlB*, *cdtB* and *ttsA,* wherein the bacterium is derived from *Salmonella* spp.

By "non-natural bacterium or bacteria" we mean a bacterial (prokaryotic) cell that has been genetically modified or "engineered" such that it is altered with respect to the naturally occurring cell. Such genetic modification may for example be the incorporation of additional genetic information into the cell, modification of existing genetic information or indeed deletion of existing genetic information. This may be achieved, for example, by way of transfection of a recombinant plasmid into the cell.

By "inactivating mutations", we mean modifications of the natural genetic code of a particular gene or gene promoter associated with that gene, such as modification by changing the nucleotide code or deleting sections of nucleotide or adding non-coding nucleotides or non-natural nucleotides, such that the particular gene is either not transcribed or translated appropriately or is expressed into a non-active protein such that the gene's natural function is abolished or reduced to such an extent that it is not measurable. Thus, the mutation of the gene inactivates that gene's function or the function of the protein which that gene encodes.

As would be understood by a person of skill in the art, genes may be mutated by a number of well-known methods in the art, such as homologous recombination with recombinant plasmids targeted to the gene of interest. In which case an engineered gene with homology to the target gene is incorporated into an appropriate nucleic acid vector (such as a plasmid or a bacteriophage), which is transfected into the target cell. The homologous engineered gene is then recombined with the natural gene to either replace or mutate it to achieve the desired inactivated mutation. Such modification may be in the coding part of the gene or any regulatory portions, such as the promoter region. As would be understood by a person of skill in the art, any appropriate genetic modification technique may be used to mutate the genes of interest, such as the CRISPR/Cas system, e.g. CRISPR/Cas 9.

Thus, numerous methods and techniques for genetically engineering bacterial strains will be well known to the person skilled in the art. These techniques include those required for introducing heterologous genes into the bacteria either via chromosomal integration or via the introduction of a stable autosomal self-replicating genetic element. Exemplary methods for genetically modifying (also referred to as "transforming" or "engineering") bacterial cells include bacteriophage infection, transduction, conjugation, lipofection or electroporation. A general discussion on these and other methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); which are hereby incorporated by reference.

It is envisaged that inactivating mutations (e.g. deletions) in the genes *ptlA*, *ptlB* and *cdtB* will prevent the *Salmonella* spp. from producing the typhoid toxin and that inactivating mutations (e.g. deletions) in *ttsA* will prevent the *Salmonella* spp. from secreting the typhoid toxin.

The non-natural bacterium of the invention is derived from *Salmonella* spp. It is envisaged that the non-natural bacterium may be derived from *Salmonella enterica*, in particular. It is particularly preferred that the non-natural bacterium is derived from *S. enterica* Typhi, *S. enterica* Paratyphi A, *S. enterica* Paratyphi B or *S. enterica* Paratyphi C. The genome sequences of these *Salmonella* spp. are well known to a person of skill in the art.

The non-natural bacteria of the invention will provide live vaccines against typhoid and paratyphoid (enteric) fever, in addition to vectors for the delivery of heterologous antigens to humans as vaccines for other diseases, as explained further below.

In certain embodiments of the present invention the one or more inactivating mutations comprise the partial or complete deletion of the one or more genes (cistrons) selected from *ptlA*, *ptlB*, *cdtB* and *ttsA.* Thus, in these embodiments, the gene(s) of interest is mutated such that all or a large part of its sequence is removed from the bacterium. This may be achieved for example using homologous recombination, as would be understood by a person of skill in the art.

In certain embodiments of the present invention, the one or more inactivating mutations prevent the expression of said one or more genes. Thus, rather than partially or fully removing the genes from the bacterium, these mutations prevent translation of the genes into mRNA, thereby disrupting the production of Typhoid toxin. One method by which these inactivating mutations may be achieved is by modifying (e.g. partially or full deleting, or mutating) the promoters operably linked to said one or more genes.

The nucleotide sequence of the *pltA* cistron is provided below as (SEQ ID NO: 1):

atgaaaaagttaatattcttaaccttatctatagttagctttaataactatgctgtagattttgtgtatcgtgtggactca
accccgccggacgttatttttcgcgatgggttttcactacttgggtataaccgtaactttcagcaatttattagtggaa
ggtcatgtagtggtggaagtagtgacagtcgctatattgcaacaacctcaagtgttaatcaaacatatgctatagc
cagagcgtactattctcgctcaacattcaaaggtaatttatacagatatcagattcgtgcagataataatttctaca
gcttgctcccatccatcacctatctggaaacgcaaggtggtcactttaatgcttatgaaaaaacgatgatgcgatt
gcaaagagagtatgtttccacattatctattttacccgagaatattcaaaaggccgtggcgctagtttatgatagcg
caaccggtctagtaaaggatggtgtaagcacaatgaatgccagttatttaggtttaagcactacgtctaatcctgg
tgtgatacctttttcttccggaaccgcagacgtatacccaacaacgaattgatgcattcggcccattaataagttcat
gcttttcaataggtagcgtatgtcattcacatcgagggcaaagagctgacgtatacaacatgtctttttatgatgca
agacctgtaatagaacttatactttctaaataa

The nucleotide sequence of the *pltB* cistron is provided below as (SEQ ID NO: 2):

atgtatatgagtaagtatgtacctgtttatacattattaattctcatttattcttttaatgccagcgctgagtggacagga
gataatacgaacgcctactactcagacgaagttatcagtgaattacatgttggtcagatagatactagtccttatttt
tgcataaaaacggttaaagctaacggtagcggtacaccagttgttgcatgtgcggtatcaaagcagagcatatg
ggcgccctcctttaaagaacttcttgatcaggcaagatatttttacagtacagggcaatccgtaaggattcatgttc
aaaaaaatatctggacctatccgctttttgttaatacctttcagcaaatgctcttgtgggactatcatcgtgcagtgc
gacacaatgctttggacccaagtaa

The nucleotide sequence of the *cdtB* cistron is provided below as (SEQ ID NO: 3):

atgaaaaaacctgtttttttccttctgaccatgatcatctgcagctatatttcttttgcctgcgctaatatcagtgactac
aaagttatgacctggaatcttcagggctcttcagcatctacagaaagtaaatggaatgtcaatgtcagacagcttt
taagtggtactgccggtgtggatattcttatggtacaggaggccggtgctgttcccacctcagcggttcctaccgg
acgacatattcagccttttggagtgggtattcccattgatgaatacacctggaatctcggaaccaccagccgtca

ggatataagatatatctaccactcggctattgatgttggagcacgccgtgttaatctggcaatagtttccagacaa
agagcggataatgtttatgtcttgcgtccgacaactgtcgcatctcgccccgtcattggcatcggactgggtaatg
atgtttttctgacagcgcacgcactggctagtggaggtccggatgctgcagctattgtcagggttaccattaattttt
tagacaacctcagatgcggcatttatcctggtttcttgccggggattttaatcgcagcccagacagacttgaaaat
gacctgatgactgagcatctggaacgagttgtagccgtactcgcacctacagaacccacgcaaattggcggtg
gtattttagattatggggtcattgtcgatcgagcaccttattcacaaagggttgaagcattacgtaatccacaactc
gcttctgatcattatcccgtagcctttttggcacgaagctgttaa

The nucleotide sequence of the *ttsA* cistron is provided below as (SEQ ID NO: 4):

atgactaaagatgaaatctttgcagctatccttagtagagaaggcggttacgttgatcaccctgacgacagggg
ggggccaacgcattggggtataaccctgacgacggcccgggctaatggctatatgggggatatgcggaatctt
acccgtaatcaagcattaaaaatccttgaggcagattattggtatggtccacgacttgatcaggttgccattatctct
cactctattgcagctgagctttgtgatactggtgtgaacatgggaccctcaatcccgattaagtacttccagcgttg
gcttaatgtttttaatgatcagcaaaaaatttatccggatttgatagcagatggtcagattgggccacggacgctttc
ggcactaacatttttctctctcaccggagagatgagggcgaaatgatacttatccgggcattgaattgtagtcag
ggacaacgctatctggagttagctgaaaagcgccaggcgaatgaatcatttgtttatggatggataaaggaac
gggtaagattgtaa

In some embodiments of the present invention, the non-natural bacterium is further engineered to express one or more genes encoding one or more heterologous antigens.

As used herein "heterologous gene" refers to a gene that has been introduced into the bacterium, i.e. the introduction of a gene that was not previously present. The gene may be endogenous or exogenous to the bacterium, whereby these terms have their normal meaning in the art. For an endogenous gene, this may comprise introducing an additional copy or copies of said one or more endogenous genes in a heterologous manner. The endogenous gene or genes may also comprise introducing dominant variants of said gene or genes into the host bacterium, whereby "dominant" refers to the ability of the heterologous gene to functionally out-compete the naturally-occurring endogenous counterpart.

By "heterologous antigen" we mean either the protein product encoded by a heterologous gene or another heterologous antigen, as required (for example a nucleic acid or polysaccharide). Thus, as used herein, an "antigen" refers to any molecule that is capable of inducing an immune response in a host.

The heterologous antigen may be under to control of a constitutive, spatially-restricted, or inducible promoter. The inventors have surprisingly found that the mutation of genes required for expression of the Typhoid toxin attenuates the bacterium and improves immunogenicity. Thus, the non-natural bacterium of the invention is an effective vaccine carrier.

The ability of the non-natural bacterium to induce an immune response against an expressed heterologous antigen can be tested by, for example, immunising animal hosts such as mice. The bacterial vaccine can be administered via, for example, the orogastric, intranasal or intraperitoneal routes. The bacteria then invade or are phagocytosed by antigen-presenting cells which induce antigen expression, leading to the generation of an immune response against said antigen by presentation via Major Histocompatibility Complex (MHC) Class I and II pathways. Numerous tests of this process and the concomitant immunity are known to the skilled person.

It is envisaged that the heterologous antigen may be expressed by the non-natural bacterial cell of the invention and the resultant protein antigen presented by antigen presenting cells (APCs) that process the bacteria in the body of a patient to be treated as outlined below. Alternatively, it is envisaged that the heterologous antigen may remain encoded in nucleic acid (e.g. DNA or RNA) in the non-natural bacterial cell of the invention and then expressed by the cellular machinery in the APC of the patent to be treated. Thus, the present invention envisages delivery of the heterologous antigen to a patient to be vaccinated as a peptide or as a DNA/RNA vaccine.

Thus, as indicated above, while the heterologous antigen may be a polypeptide expressed in the bacterium by a heterologous gene, it may alternatively or additionally be the heterologous gene itself or another transfected molecule, such as a polysaccharide. Thus, the heterologous antigen is any molecule introduced into the non-natural bacterium that is capable of inducing a protective immune response in a patient administered the non-natural bacterium of the invention. It is envisaged that the bacterium will be taken up by APCs on administration to the patient and then processed and presented to the patient's immune system. Thus, this may be directly processing heterologous polypeptide antigen expressed in the bacterium or heterologous polysaccharide or DNA present in the bacterium. Or, the heterologous DNA in the bacterium may be transcribed and/or translated in the APC or other host (patient) cell to provide peptides for presentation (i.e. DNA vaccine).

In some embodiments, the heterologous antigen is derived from a pathogenic organism selected from the group consisting of virus, bacterium, fungus, or other eukaryotic parasite.

Wherein the heterologous antigen is derived from a virus, the heterologous antigen may be derived from a virus selected from the list consisting of Dengue Fever Virus, Ebola Virus, Encephalomyocarditis Virus, Hepatitis A, B and C Viruses, Herpes Simplex Virus, Human Enterovirus, Human Immunodeficiency Virus, Human Papilloma Virus, Human Parvovirus, Human Rhinovirus, Influenza Virus, Lymphocytic Choriomeningitis Virus, Middle East Respiratory Syndrome Virus, Mouse Mammary Tumour Virus, Rabies Virus, Respiratory Syncytial Virus, Severe Acute Respiratory Syndrome Coronavirus, West Nile Virus, Zika Virus or a combination thereof. Conventional heterologous antigens derived each of these viruses capable of inducing immune responses in a host are well known to the skilled person.

Wherein the heterologous antigen is derived from a bacterium, the heterologous antigen may be derived from a bacterium selected from the list consisting of *Aerococcus* spp., *Aeromonas hydrophila, Actinobaculum schaalii, Acinetobacter baumannii, Aggregatibacter actinomycetemcomitans, Alloscardovia omnicolens, Anaplasma phagocytophilum*, *Bacillus* spp. (including *B*. *anthracis* and *B*. *cereus*), *Bacteroides fragilis*, *Bartonella* spp. (including *B. henselae*), *Bordetella* spp. (including *B. pertussis*), *Borrelia* spp. (including *B. burgdorferi*), *Brucella* spp. (including *B. abortus*), *Burkholderia* spp. (including *B. cepacia*), *Campylobacter jejuni*, *Chlamydia* spp. (including *C. trachomatis*), *Chlamydophila pneumoniae*, *Clostridium* spp. (including *C. botulinum*, *C. difficile* and *C. tetani*), *Corynebacterium diptheriae*, *Enterobacter* spp. (including *E. aerogenes* and *E*. *cloacae*), *Enterococcus* spp. (including *E*. *faecalis* and *E*. *faecium*), *Escherichia coli* (including ETEC and O157:H7), *Francisella tularensis*, *Haemophilus influenzae*, *Helicobacter pylori, Klebsiella pneumoniae, Legionella pneumophila, Leptospira* spp., *Listeria monocytogenes*, *Moraxella catarrhalis*, *Mycobacterium* spp. (including *M. avium*, *M. bovis* and *M. tuberculosis*), *Mycoplasma pneumoniae*, *Neisseria* spp. (including *N. gonorrhoeae* and *N. meningitidis*), *Neoehrlichia mikurensis*, *Nocardia* spp., *Proteus mirabilis*, *Pseudomonas* spp. (including *P. aeruginosa* and *P. stutzeri*), *Rickettsia* spp., *Salmonella* spp. (including *S. enterica*), *Serratia marcescens*, *Shigella* spp. (inclusing *S. dysenteriae*, *S. flexneri* and *S. sonnei*), *Staphylococcus* spp. (including *S. aureus* and *S. epidermis*), *Stenotrophomonas maltophilia, Streptococcus* spp. (including *S. pyogenes*), *Treponema pallidum*, *Tropheryma whipplei*, *Ureaplasma urealyticum*, *Vibrio cholera*, *Yersinia* spp. (including *Y. pestis* and *Y. pseudotuberculosis*). or combinations thereof. Conventional heterologous antigens derived each of these bacteria capable of inducing immune responses in a host are well known to the skilled person.

Wherein the heterologous antigen is derived from a fungus, the heterologous antigen may be derived from a fungus selected from the list consisting of *Aspergillus* spp. (including *A. niger*), *Blastomyces* spp., *Candida* spp. (including *C. albicans, C. auris* and *C*. *tropicalis*), *Coccidioides immitis, Cryptococcus neoformans*, *Gibberella moniliformis*, *Histoplasma capsulatum, Fonsecae pedrosoi*, *Fusarium oxysporum*, *Microsporum* spp., *Pneumocystis jirovecii*, *Rhizopus oryzae* and *Trichophyton* spp. Other eukaryotic pathogens include *Acanthamoeba* spp., *Ascaris lumbricoides, Babesia* spp., *Bruga* spp. (including *B. malayi* and *B. timori*), *Cryptosporidium parvum*, *Entamoeba histolytica*, *Giardia lamblia*, *Leishmania* spp. including (*L. donovani* and *L. major*), *Necator americanus*, *Parachlamydia acanthamoebae*, *Plasmodium* spp. (including *P. falciparum* and *P. vivax*) *Toxoplasma gondii*, *Trichinella spiralis*, *Trichomonas vaginalis*, *Trypanosoma* spp. (including *T. cruzi* and *T. brucei*) and *Wuchereria bancrofti.* Conventional heterologous antigens derived each of these fungi capable of inducing immune responses in a host are well known to the skilled person.

In some embodiments, the heterologous antigen is a cancer vaccine. Exemplary cancer antigens include tumour-specific proteins, tumour-associated proteins, growth factors or angiogenesis factors. In some embodiments, the cancer antigen may be selected from the list consisting of AFP (alpha-fetoprotein), CA-125 (ovarian cancer antigen 125), CEA (carcinoembryonic antigen), endoglin (CD105; including transforming growth factor-beta receptor: TGF-beta), ETA (epithelial tumour antigen), Her2/neu (receptor tyrosine-protein kinase erbB-2), HMW-MAA (human high molecular weight-melanoma-associated antigen), ISG15 (interferon-stimulated gene 15), MAGE (melanoma-associated antigen), MUC1 (mucin 1, cell surface associated), NY-ESO-1 (New York esophageal squamous cell carcinoma 1), p53, p60, PSA (prostate-specific antigen), survivin (an apoptosis protein), TRP2 (tyrosinase-related protein 2), VEGFR2 (vascular endothelial growth factor receptor 2), or antigenic fragments thereof. As defined herein, "antigenic fragment" refers to a portion of the antigen that is capable of inducing an immune response in the host.

It is envisaged that the one or more genes encoding heterologous antigens may be chromosomally integrated or encoded by an autonomous genetic element. Examples of self-replicating autosomal genetic elements (also referred to herein as "vectors" or "replicons") include, but are not limited to, plasmids, to which the one or more heterologous genes may be attached so as to bring about the replication of the heterologous gene or attached segment in the target cell. Suitable genetic elements include those that enable the "stable" replication of the genetic element during cell division, i.e. stable genetic elements are those that allow the genetic element to be maintained in all or predominantly all progeny during successive rounds of cell division.

Suitable genetic elements will vary depending on the identity of the host bacterium from which the non-natural bacterium is derived. Examples of suitable genetic elements include, but are not limited to, plasmids comprising an origin of replication selected from pMB1, ColEl, p15A, pSC101 and PK2RK2. Other suitable origins of replication will be well known to the person skilled in the art.

The "genetic elements" described herein comprise a DNA coding sequence operably linked to a promoter. "Operably linked" refers to a juxtaposition wherein the components so described are in a relationship permitting them to function in their intended manner. For instance, a promoter is operably linked to a coding sequence if the promoter affects its transcription or expression.

In certain embodiments, the one or more genes encoding said heterologous antigens may be operably linked to an inducible promoter activated when the bacterium is internalised into a eukaryotic cell. It is envisaged that the eukaryotic cell will be an antigen presenting cell (APC), such as a dendritic cell, macrophage, B cell or other appropriate APC, as would be understood by a person of skill in the art.

As used herein, a "promoter" refers to a DNA regulatory region capable of binding RNA polymerase and initiating transcription of a downstream (3' direction) coding or non-coding sequence. A promoter may also be a regulatory DNA sequence that affects the binding of RNA polymerase at the transcription initiation site. For the purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence may be found a transcription initiation site, as well as protein binding domains responsible for the binding of RNA polymerase.

Promoters can be constitutively active (wherein "active" means transcription is "on"), spatially restricted or inducible. As used herein "spatially restricted" refers to a promoter that is only active in a specific subset of cells or cellular compartment of a multicellular organism. A spatially restricted promoter can thus be used to activate the expression of a nucleic acid in a particular tissue or cell type of a multicellular organism.

As used herein an "inducible promoter" refers to a promoter that enables the temporal and/or spatial activation of transcription in response to external physical or environmental stimuli. Inducible promoters include those activated by the presence of specific small molecules that alleviate transcriptional repression. For example, transcription from such an inducible promoter may be regulated by a "repressor protein". As used herein, "repressor protein" refers to a polypeptide that binds to and occupies the inducible promoter to prevent transcription initiation. When bound to the promoter, said repressor protein can prevent binding or recruitment of RNA polymerase or associated co-factors to the transcription initiation site to prevent the activation of transcription. However, upon binding its relevant small molecule, or encountering its relevant physical or environmental stimulus, the repressor protein can no longer bind to the promoter sequence, and thus transcriptional repression is relieved. Examples of repressor proteins include lambda repressor cl and variants thereof (e.g. the thermo-labile mutant cl857, the arabinose repressor AraC and the tetracycline repressor TetR (Bertram &Hillen (2008) Microbial Biotechnol 1:2-16; Rosano & Ceccarelli (2014) Frotniers Microbiol 5: 172; Love et al (1996) Gene 176:49-53).

Other types of inducible promoters are those that activate transcription in response to environmental stimuli. Examples of such inducible promoters include *P_{pagC}*, *P_{nirB}*, *P_{ssaG}*, *P_{sifA}*, *P_{sifB}*, *P_{sseA}*, *P_{sseG}* and *P_{sseJ}* (Dunstan et al. (1999) Infect. Immun. 67: 5133-5141; Xu et al. (2010) Infect. Immun. 78: 4828-4838; Kröger et al. (2013) Cell Host & Microbe 14: 683-695). Suitable inducible promoters for use in the invention are those that activate transcription in response to the environmental conditions of phagocytic vesicles within a eukaryotic cell, preferably wherein the eukaryotic cell is an antigen presenting cell (APC). For example, such suitable inducible promoters may activate expression of their operably linked genes at a pH in the range of pH 3 to pH 6.5, preferably at a pH in the range of pH 5.0 to pH 6.5, more preferably at a pH of about pH 5.8. The exact inducible promoter most relevant to particular embodiments of the invention will depend on the host bacterium from which the non-natural bacterium is derived. A suitable host-specific inducible promoter can be identified by culturing the host bacterium in so called "InSPI2" conditions, which are fully described in Kröger et al. 2013, Cell Host & Microbe 14: 683-695, incorporated herein by reference. InSPI2 conditions comprise culturing the bacterium in Phosphate Carbon Nitrogen (PCN) medium supplemented with 0.4 mM inorganic phosphate at pH 5.8. PCN medium is a medium designed to induce phagosome-dependent promoters, as described in Löber et al. 2006, Int. J. Med. Microbiol. 296: 435-447, which is hereby incorporated by reference. The bacterium can be cultured until an OD₆₀₀ₙₘ of 0.3 is reached. Deep sequencing and RNA-sequencing of mRNA transcripts can then be combined to ascertain which genes are most preferentially expressed under InSPI2 conditions in the bacterium. When compared with the transcriptome analysis of the bacterium under standard laboratory conditions, such an analysis can be used to reveal inducible promoters most selectively activated under conditions reminiscent of phagocytic vesicles of APCs.

Thus, it is envisaged that the one or more heterologous genes of the invention may be under the control of an inducible promoter selected from *P_{pagC}*, *P_{nirB}*, *P_{ssaG}*, *P_{sifA}*, *P_{sifB}*, *P_{sseA}*, *P_{sseG}* and *P_{sseJ}* or a combination thereof. Transcription from all of *P_{pagC}*, *P_{nirB}*, *P_{ssaG}*, *P_{sifA}*, *P_{sifB}*, *P_{sseA}*, *P_{sseG}* and *P_{sseJ}* is active in phagosomes. The invention embodies variants and fragments of said inducible promoters that maintain inducible operability within phagosomes, or under InSPI2 conditions. In some embodiments, the functional variants include those having similar or modified sequences to *P_{pagC}*, *P_{nirB}*, *P_{ssaG}*, *P_{sifA}*, *P_{sifB}*, *P_{sseA}*, *P_{sseG}* and *P_{sseJ}*, and similar or substantially identical promoter activity as the wild-type promoter from which the variant is derived, particularly with respect to its ability to induce expression *in vivo.* Similar modified sequences may include having at least about 70%, 80%, 85%, 90%, 95%, 96%, 97%, 98% or 99% identity to the wild-type sequence of any of *P_{pagC}*, *P_{nirB}*, *P_{ssaG}*, *P_{sifA}*, *P_{sifB}*, *P_{sseA}*, *P_{sseG}* and *P_{sseJ}.*

It is preferred that where the heterologous antigen is a recombinant protein expressed in a *Salmonella enterica* vector (vaccine) of the invention, that the expression of the antigen in the vector is controlled by a promoter selected from *P_{pagC}*, *P_{nirB} P_{ssaG}*, *P_{sifA} P_{sifB}*, *P_{sseA}*, *P_{sseG}*, and *P_{sseJ}*.

Further, where the heterologous antigen is a DNA molecule (DNA vaccine) to be expressed in a human patient's cell, it is preferred that the promoter controlling its expression includes P_{CMV}.

In some embodiments, the non-natural bacterium of the invention may further comprise attenuating mutations in one or more essential genes selected from *aroA, aroC, ssaV* or a combination thereof. It is preferred that the attenuating mutations are basal whereby "basal" refers to the bacterium already comprising the attenuating mutation within the endogenous gene. The attenuation of the essential gene(s) can be achieved by excising the chromosomal gene, or introducing attenuating mutations into the coding sequence of the essential gene, or by repressing the expression of the essential gene. In some embodiments, the host bacterium will comprise basal attenuating mutations or deletions of the one or more essential genes. In some embodiments, the one or more essential genes may be additionally selected from *purA, purE, asd, cya, crp*, *clpX*, *galE, guaA, guaB, htrA, msbB*, *phoP, phoQ*, *pmi, pur, ssaJ, ssaK, ssaU, ssaL, ssaM, ssaO, ssaP, ssaQ, ssaR, ssaS, ssaT, ssaD, ssaE, ssaG, ssal, ssaC,* and *ssaH.* The essentiality of each of these genes is described in WO 2009/158240, which is hereby incorporated by reference.

In a further aspect, the present invention provides a non-natural bacterium according to the first aspect for use in the treatment or prevention of a disease. The non-natural bacterium is thus provided for administration to a patient in need thereof. It is envisaged that the patient will be a mammal. Preferably the mammal is a human.

Alternatively, veterinary uses are also included. For example, the mammal may be a farm animal or companion animal, for example a cow, sheep, horse, pig, cat, dog, rabbit, mouse or rat.

Preferably the disease according to any aspect herein is an infectious disease or cancer.

Thus, the present invention provides the non-natural bacterium for use according to the preceding aspect, wherein the bacterium is for use in vaccination against a disease.

The disease may be a disease caused by *Salmonella* spp., for example the disease may be typhoid or paratyphoid enteric fever. In this embodiment, the *Salmonella* spp. with the inactivating mutation in one or more of the genes *ptlA*, *ptlB, cdtB* and *ttsA* may be directly used as a live vaccine against typhoid or paratyphoid enteric fever in a patient in need thereof.

Alternatively, of course, the non-natural *Salmonella* spp. bacterium of the invention may be for use in treating or preventing any disease according to the heterologous antigen that it expresses. Thus, the non-natural bacterium may be for use in treating or preventing an infection in a patient from any organism listed above as the possible source of the heterologous antigen. In this embodiment, the *Salmonella* spp. bacterium may thus initially be modified so as to contain the inactivating mutation in one or more of the genes *ptlA*, *ptlB*, *cdtB* and *ttsA* and then subsequently transformed with the heterologous gene by an appropriate mechanism. When administered to the patient, the bacterial cell is intended to be phagocytosed by an appropriate APC and the heterologous gene expressed in the bacterial cell will then be processed by the APC and presented to the immune system of the patient to be treated or vaccinated.

In some embodiments, the disease to be treated or prevented may be cancer. The cancer may be selected from the list consisting of Adenoid Cystic Carcinoma, Adrenal Gland Tumour, Amyloidosis, Anal Cancer, Ataxia-Telangiectasia, Atypical Mole Syndrome, Beckwith Wiedemann Syndrome, Bile Duct Cancer, Birt Hogg Dube Syndrome, Bladder Cancer, Bone Cancer, Brain Tumour, Breast Cancer, Carcinoid Tumour, Carney Complex, Cervical Cancer, Colorectal Cancer, Ductal Carcinoma, Endometrial Cancer, Oesophageal Cancer, Familial-Adenomatous Polyposis, Gastric Cancer, Gastrointestinal Stromal Tumour, HER2-Positive Breast Cancer, Hereditary Prostate Cancer, Islet Cell Tumour, Juvenile Polyposis Syndrome, Kaposi's Sarcoma, Kidney Cancer, Laryngeal Cancer, Acute Lymphoblastic Leukaemia, Acute Myeloid Leukaemia, Chronic Lymphocytic Leukaemia, Chronic Myeloid Leukaemia, Acute Lymphocytic Leukaemia, Liver Cancer, Lobular Carcinoma, Lung Cancer, Small Cell Lung Cancer, Hodgkin's Lymphoma, Non-Hodgkin's Lymphoma, Lynch Syndrome, Malignant Glioma, Mastocytosis, Melanoma, Meningioma, Multiple Endocrine Neoplasia Type 1, Multiple Endocrine Neoplasia Type 2, Multiple Myeloma, Myelodysplastic Syndrome (MDS), Nasopharyngeal Cancer, Neuroendocrine Tumour, Nevoid Basal Cell Carcinoma Syndrome, Oral Cancer, Osteosarcoma, Ovarian Cancer, Pancreatic Cancer, Pancreatic Neuroendocrine Tumours, Parathyroid Cancer, Penile Cancer, Peritoneal Cancer, Peutz-Jeghers Syndrome, Pituitary Gland Tumour, Pleuropulmonary Blastoma (Childhood), Polycythemia Vera, Prostate Cancer, Renal Cell Cancer, Retinoblastoma (Childhood), Salivary Gland Cancer, Sarcoma, Sarcoma - Alveolar Soft Part and Cardiac, Sarcoma - Kaposi, Skin Cancer (Non-Melanoma), Small Bowel Cancer, Small Intestine Cancer, Small Intestine Cancer, Stomach Cancer, Testicular Cancer, Thymoma, Thyroid Cancer, Turcot Syndrome, Uterine (Endometrial) Cancer, Vaginal Cancer, Von-Hippel-Lindau Syndrome and Wilms' Tumour (Childhood).

In some embodiments, the heterologous gene encoding the tumour antigen is cognate to the cancer to be treated. In other words, the heterologous tumour antigen can be a tumour-specific antigen expressed in the tumour cells of the cancer to be treated. In some embodiments, the heterologous tumour antigen is a tumour-associated antigen (TAA), whereby a "TAA" refers to an antigen that is expressed at elevated levels in the tumour cells.

Thus, in a further aspect, the present invention provides a vaccine composition comprising the non-natural bacterium of the first aspect and a pharmaceutically acceptable carrier. It is preferred that the vaccine composition will be formulated for oral or parenteral delivery. The compositions may be in the form of capsules, lozenges, liquid or gel preparations, such as oral or sterile parenteral solutions or suspensions. Capsules for oral administration may be in unit dose presentation form, and may contain conventional excipients such as binding agents, for example syrup, acacia, gelatin, sorbitol, tragacanth, cholestyramine or polyvinylpyrrolidone, fillers for example lactose, sugar, maize-starch, calcium phosphate, sorbitol or glycine, tabletting lubricant, for example magnesium stearate, talc, polyethylene glycol or silica, disintegrants for example potato starch, or acceptable wetting agents such as sodium lauryl sulphate. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats, emulsifying agents, for example lecithin, sorbitan monooleate, or acacia, non-aqueous vehicles (which may include edible oils), for example almond oil, fractionated coconut oil, oily esters such as glycerine, propylene glycol, or ethyl alcohol, preservatives, for example methyl or propyl p-hydroxybenzoate or sorbic acid, and if desired conventional flavouring or colouring agents. The vaccine composition may also be administered parenterally in a sterile medium. Depending on the vehicle and concentration used, the vaccine composition can either be suspended or dissolved in the vehicle. It is envisaged that the parenteral route, such as intravenous or intramuscular delivery, may be particularly preferred for the delivery of anti-cancer vaccine compositions.

The present invention also provides a method of preventing or treating a disease according to the earlier aspects, comprising administering a non-natural bacterium or vaccine composition according to the earlier aspects to a patient in need thereof.

The invention also provides a method of manufacturing a non-natural bacterium according to any of the preceding aspects, comprising introducing into the non-natural bacterium any of the genetic modifications listed herein using techniques well known to a person of skill in the art.

For the avoidance of doubt, all embodiments listed above apply to all aspects of the invention provided above.

In order that the invention may be more clearly understood embodiments thereof will now be described by way of example with reference to the accompanying figures.

### Example 1

Polymerase chain reaction (PCR) primer pairs were designed with 5' ends having 50 bp of homology to regions flanking each of the target genes (Figure 1), and 3' ends which annealed either side of a chloramphenicol acetyltransferase gene *cat* which is flanked by *E*. *coli dif* sites, such that the resulting PCR products have 5' and 3' ends homologous to the proximal and distal ends of the target gene or genes to be deleted, and an internal *cat* gene being flanked by *dif* sites. The template plasmid for the PCR reactions was pDifCat2TOPO. This was constructed using the primers 5Notdifcat2 (5'-taagcggccgcatttaacataatatacattatgcgcaccatccgcttattatcacttat-3') and 3Notdifcat2 (5'-tccgcggccgcggtgcgcataatgtatattatgttaaatcccttatgcgactcctgc-3') to amplify a synthesised *cat* gene from the plasmid pMKtetORTDAP. The *dif-cat-dif* PCR product of 1048 bp was then gel-purified and cloned into a pCR-Blunt plasmid to create pDifCat2TOPO.

The primers to generate the gene deletion cassettes are shown in Table 1. PCR reactions were carried out to generate the gene deletion cassettes using PLTB5 plus one of the other primers; these were purified by agarose gel electrophoresis and transformed into *S. Typhi* DTY8 (Ty2 *aroC*) containing the plasmid pLGBEk, which carries the *bet*, *exo* and *gam* genes conferring the lambda Red homologous recombination functions (Murphy 1998, J. Bacteriol. 180: 2063-2071) and the kanamycin phosphotransferase gene *kan.* Single colonies were selected on LB agar containing 20 µg/ml chloramphenicol, and subsequently cultured in LB broth in the absence of chloramphenicol to enable excision of the *cat* gene by the Xer-cise mechanism (Bloor & Cranenburgh 2006, Appl. Environ. Microbiol. 72: 2520-2525). Dilutions were prepared and plated to obtain further single colonies, which were screened for antibiotic sensitivity (to chloramphenicol and kanamycin) to confirm the loss of the *cat* gene and pLGBk helper plasmid. Chromosomal target loci were amplified by PCR and sequenced to confirm the deletions.

**Table 1. Primers containing dif sites (bold) used to delete genes involved in typhoid toxin structure and secretion. Chromosomal target site homology is underlined.**

| **Primer name** | **Sequence (5'→3')** | **SEQ ID NO** |
|---|---|---|
| PLTB5 | tataatgaatttgtgttttattaattattttaaacgtacaggagagtagtggtgcgcataatg tatattatgttaaatcccttatgcgactcctgc | SEQ ID NO: 5 |
| PLTA3 | aactaataaacgggctgtttaatttaataggcaacataggtaagtttcatatttaacataat atacattatgcgcaccatccgcttattatcacttat | SEQ ID NO: 6 |
| TTSA3 | atatcattatatgctatcatcacaaaccgctatagtggtaagtaagggaaatttaacata atatacattatgcgcaccatccgcttattatcacttat | SEQ ID NO: 7 |
| CDTB 3 | aacacatatatcattcagataaagaagtaatgctcgggagagtagatattatttaacata atatacattatgcgcaccatccgcttattatcacttat | SEQ ID NO: 8 |

The resulting *S. Typhi* strains were therefore deleted of i) *pltA* and *pltB*; ii) *pltA*, *pltB* and *ttsA*; and iii) *pltB*, *pltA*, *ttsA* and *cdtB* (in addition to theoretical cistron *sty 1887;* Hodak & Galán 2013, EMBO Reports 14: 95-102).

### References

Bloor & Cranenburgh 2006, Appl. Environ. Microbiol. 72: 2520-2525.
Garmory et al. 2002, FEMS Microbiol. Rev. 26: 339-353.
Grant et al. 2016, Infect. Immun. 84: 989-997.
Guidi et al. 2013, Cellular Microbiol. 15: 2034-2050.
Hodak & Galán 2013, EMBO Reports 14: 95-102.
Kochi et al. 2003, Expert Rev. Vaccines 2:31-43.
Kotton & Hohmann 2004, Infect. Immun. 72: 5535-5547.
McIntyre et al. 2014, Plos One 9: e103529.
Murphy 1998, J. Bacteriol. 180: 2063-2071.
Song et al. 2013, Nature 499: 350-354.

## Claims

1. A genetically engineered non-natural bacterium comprising inactivating mutations in one or more genes selected from *ptlA*, *ptlB*, *cdtB* and *ttsA,* wherein the bacterium is derived from *Salmonella* spp.

2. The non-natural bacterium of claim 1, wherein the bacterium comprises Salmonella enterica, preferably wherein the bacterium is selected from *S. enterica* Typhi, *S. enterica* Paratyphi A, *S. enterica* Paratyphi B or *S. enterica* Paratyphi C.

3. The non-natural bacterium of any preceding claim, wherein the one or more inactivating mutations comprise the partial or complete deletion of the one or more genes selected from *ptlA*, *ptlB*, *cdtB* and *ttsA.*

4. The non-natural bacterium of any preceding claim, wherein the one or more inactivating mutations prevent the expression of said one or more genes.

5. The non-natural bacterium of claim 4, wherein the inactivating mutation comprises modifying the promoters operably linked to said one or more genes.

6. The non-natural bacterium of any preceding claim, further engineered to express one or more genes encoding one or more heterologous antigens.

7. The non-natural bacterium of claim 6, wherein the one or more genes encoding heterologous antigens are chromosomally integrated or encoded by an autonomous genetic element.

8. The non-natural bacterium of any of claims 6 or 7, wherein the one or more genes encoding said heterologous antigens are operably linked to an inducible promoter activated when the bacterium is internalised into a eukaryotic cell.

9. The non-natural bacterium of claim 8, wherein the one or more inducible promoters is selected from *P_{pagC}*, *P_{nirB}*, *P_{ssaG}*, *P_{sifA}*, *P_{sifB}*, *P_{sseA}*, *P_{sseG}*, *P_{sseJ}* or any combination thereof.

10. The non-natural bacterium of any preceding claim, further comprising attenuating mutations in one or more genes selected from *aroA* and/or *aroC* and/or *ssaV*; preferably wherein the attenuating mutations are basal.

11. The non-natural bacterium of any preceding claim, for use in the treatment or prevention of a disease, optionally wherein the disease is an infectious disease or cancer.

12. The non-natural bacterium for use according to claim 11, wherein the bacterium is for use in vaccination against a disease.

13. The non-natural bacterium for use according to claim 11 or 12, wherein the disease is typhoid or paratyphoid enteric fever.

14. A vaccine composition comprising the non-natural bacterium of any of claims 1 to 10 and a pharmaceutically acceptable carrier.

15. A method of manufacturing a non-natural bacterium according to any of claims 1 to 10, comprising introducing into the non-natural bacterium any of the genetic modifications of claims 1 to 10.
